# EUROPEAN PATENT SPECIFICATION

(11) **EP 4 142 564 B1**
(45) Date of publication and mention of the grant of the patent: **04.03.2026**
(21) Application number: 21725383.0
(22) Date of filing: 22.04.2021
(51) Int. Cl.: A61B 1/005

(54) **AN INSERTION TUBE SUB-ASSEMBLY FOR AN ENDOSCOPE**
EINFÜHRROHR-UNTERBAUGRUPPE FÜR EIN ENDOSKOP
SOUS-ENSEMBLE TUBE D'INSERTION POUR UN ENDOSCOPE

(30) Priority: 27.04.2020 DK PA202070262
(43) Date of publication of application: 08.03.2023
(73) Proprietor: Ambu A/S, 2750 Ballerup (DK)
(72) Inventor: NIEKRAWIETZ, Sonja Elke, 86199 Augsburg (DE); SCHÜTZ, Günter Wilhelm, 86152 Augsburg (DE)
(74) Representative: AWA Denmark A/S
(86) International application number: PCT/DK2021/050123
(87) International publication number: WO 2021/219179

(56) References cited:
- EP-A2- 1 545 680
- EP-B1- 1 545 680

## Description

The present disclosure relates to an insertion tube sub-assembly for an endoscope.

Endoscopes typically comprises a handle gripped by an operator and a flexible insertion tube terminated at the distal end in a highly bendable, e.g. articulated, bending section, controllable by the operator. Thus, the controllable bending section is normally an articulated section at the distal tip of the insertion tube that can be controlled by the operator via control knobs arranged on the handle, allowing the operator to advance the distal tip of the endoscope to a desired location by means of a series of actions involving *inter alia* bending the bending section in a desired direction, advancing the insertion tube and turning the insertion tube by turning the handle which is rigidly connected thereto. Negotiating a tortuous path of bends and turns to a location of interest may subject the insertion tube including the distal controllable bending section to substantial forces including compression, torsion, and bending. In fact, it can be said that it is an essential part of the purpose of the insertion tube to transmit the longitudinal pushing forces and rotary torsional forces from the handle to the distal end of the insertion tube.

Typically, the tubular main body of the insertion tube is provided as a tubular member comprising at least three layers, an outer air and/or watertight sheath of a polymer material, an inner layer comprising a wound helical member and an intermediate layer of a braided material. The wound helical member provides the tubular member with compression resistance, preventing it largely from axial compression and radial compression, thus preventing it from kinking. The braided layer on the other hand provides torsional resistance, so that a turning motion of the handle to which the insertion tube is attached is transferred to the distal end of the insertion tube. A known example is found in US2017/0010458.

JP2017-49419 discloses an endoscopic insertion portion comprising a bent tube and a flexible tube connected to each other via a threaded connection mechanism.

The bending section normally comprises a separate unit that therefore needs to be joined with the distal end of the tubular main body to form the insertion tube, typically in the form of an insertion tube sub-assembly to be assembled with the remainder of the endoscope, in particular the handle, at a later stage in the assembly process of the endoscope. Just like the rest of the insertion tube, this joint is subjected to the above-mentioned compression, torsional, and bending forces, and must be able to withstand them just as well as the rest of the insertion tube.

In medical endoscopes there is currently a trend towards disposable endoscopes, that is to say single use endoscopes that are used with one patient only, upon which the endoscope is thrown away rather than cleaned, sterilized and reused. Although cleaning and sterilization of multiple use endoscopes is somewhat costly, there is obviously a need to keep the manufacturing costs for disposable endoscopes down to make disposing of them affordable. Making the parts for disposable endoscope simple and cheap to manufacture and easy to assemble is therefore desirable. This also applies to the joint between the tubular main body and the bending section.

As for joining the separate unit forming the bending section with the distal end of the distal end of the tubular main body US2017/0010458 suggests the use of an interposed connecting member comprising two separate cylindrical members engaging the inside and the outside of the tubular main body, respectively.

Based on the above it is an object of the disclosure to provide a simple and cost-efficient way to assemble an endoscope, in particular providing a strong and reliable joint between the bending section and the insertion tube main body.

According to a first aspect of the disclosure this object is achieved by an endoscope comprising an insertion tube sub-assembly where the insertion tube sub-assembly comprises a bending section part and a tubular main part adapted to extend from an endoscope handle towards the bending section part at the distal end of the insertion tube, wherein the tubular main part comprises a wound helical member, and where the bending section part comprises a number of articulated segments, and a connection part adapted for connecting the bending section part to the tubular main part, the connection part comprising a thread adapted to engage the wound helical member of the tubular main part, wherein the external thread comprises a number of individual thread segments arranged with spaces between the individual thread segments.

By providing an external thread on the bending section part it becomes a very simple operation to join it with the insertion tube main body as the two separate parts may simply be screwed together. With the individual thread segments arranged with spaces between the individual thread segments, the joint between the insertion tube and the tubular main body of the insertion tube sub-assembly is further secured and reinforced, in particular when glue is introduced in at least one of said spaces during assembly. Furthermore, the bending section part constitutes one single item which is easy and cost-efficient to manufacture, e.g. by moulding it as one single integral piece.

According to a third aspect of the disclosure the object is achieved by a system comprising a display unit and an endoscope according to the first aspect of the disclosure connectable to said display unit.

According to yet a further preferred embodiment, the connection part comprises a generally frusto-conical outer surface on which said external thread is provided. The frusto-conical shape provides mechanical strength against external forces working on the joint when the insertion tube is introduced through a tortuous path in the body of a patient during treatment, examination or other medical procedure.

According to a preferred embodiment of the second aspect of the disclosure the bending section part is a single-piece integrally moulded part.

The disclosure will now be made in greater detail based on nonlimiting exemplary embodiments and with reference to the drawings. on which:
Fig. 1 shows a display unit and a medical system comprising an endoscope with an insertion tube sub-assembly according to the disclosure connectable to said display unit,
Fig. 2 shows a bending section part for the insertion tube sub-assembly according to the disclosure, and
Fig. 3 shows an isometric view of the tubular main body forming part of the insertion tube sub-assembly according to the disclosure with the braid and outer layer partly removed in order to better show the wound helical member.

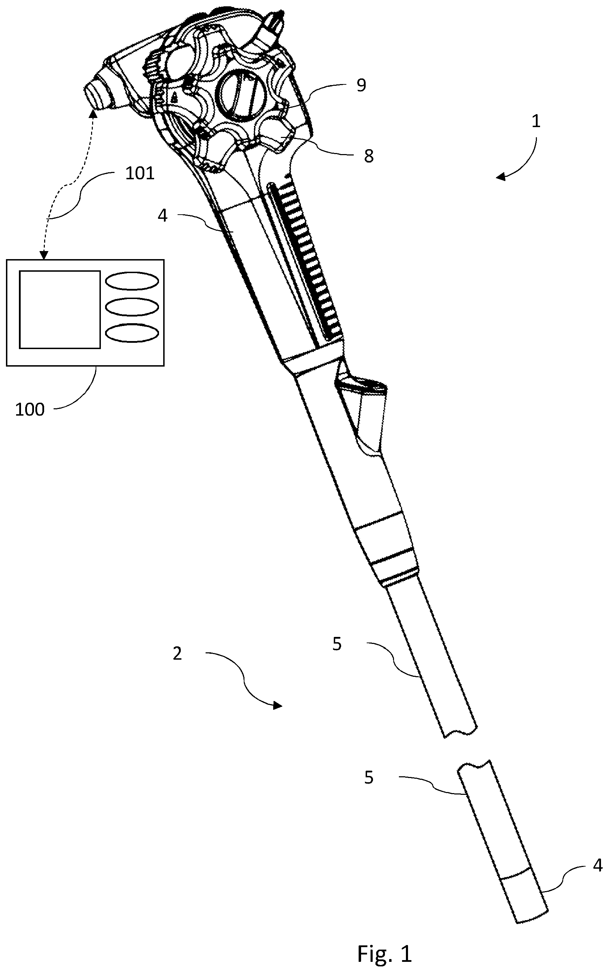

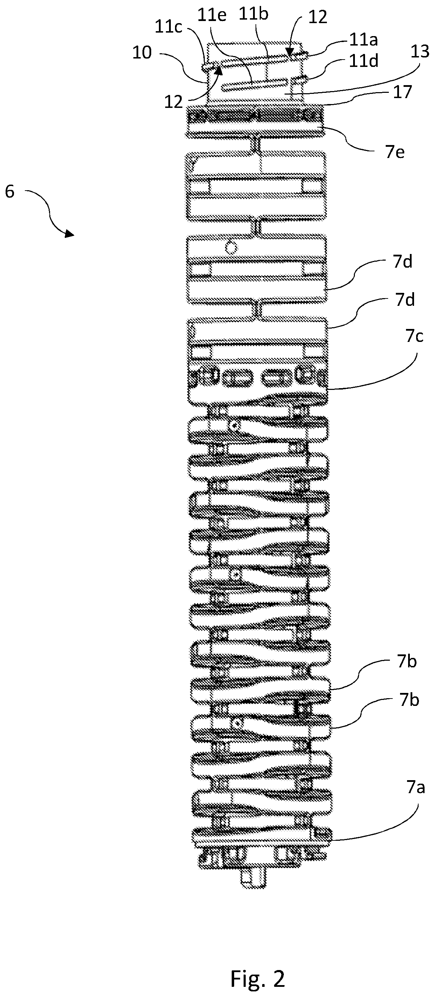

Turning first to Fig. 1 a system comprising a display unit 100 and an endoscope 1 incorporating the disclosure is shown. The endoscope 1 is connectable to the display unit via a cable 101 or a wireless connection. The endoscope 1 comprises an insertion tube 2 extending from an endoscope handle 3. At the distal end of the insertion tube 2 a bending section 4 is provided. The bending section 4 is far more bendable than the elongate tubular main body 5 of the insertion tube 2. Within the bending section 4 is a bending section part 6 shown in Fig. 2, but not visible in Fig. 1 because it is covered and protected by an outer sheath.

The bending section part 6 comprises a number of articulated segments 7a, 7b, 7c, 7d and 7e, interconnected by means of hinge members, such as foil hinges. These segments allow the bending section part 6 to bend in four directions in two perpendicular planes, i.e. up, down; left, right by means to respective control knobs 8, 9 arranged on the handle and connected to the segments 7a and 7c by means of pull-wires in a manner *per se* known, and therefore not illustrated.

As will be noticed from Fig. 2 the bending section part 6 comprises sets of different articulated intermediate segments 7b and 7d separated by an interface segment 7c. By terminating an outer sheath of a Bowden cable in the interface segment 7c, this allows for the bending section 4 to be divided into an active bending section comprising the articulated segments 7b and a passive bending section comprising the articulated segments 7d. That is to say, so that the passive bending section is between a narrower part 10 of the most proximal segment 7e and the interface segment 7c, and the active bending section is between the interface segment and the most distal segment 7a.

The intermediate segments 7b, 7d in each set may differ in immaterial details such as the presence or location of remains of moulding inlets, ejector marks etc. from manufacture, and the fact that each of them are turned by 90 degrees with respect to its neighbor. Also, as will be noted there is quite a degree of the rotational symmetry for the intermediate segments 7b, 7d within each set, such that turning an intermediate segment 7b, 7d by 90 degrees makes it correspond to its own mirror image.

As can also be seen in Fig. 2, the most proximal segment 7e comprises a narrower part 10. The narrower part 10 is preferably intergrally moulded together with remainder of the bending section 6, so that the entire bending section part 6, as shown in fig 2, is an integrally moulded single piece item. The outer surface 13 of the narrower part 10 is provided with a an external, i.e. male, thread 11a-11e. The thread 11a-11e is preferably not continuous, but segmented, so that spaces 12 exist between neighboring segments 11a-11e as seen in a circumferential direction.

The outer surface 13 is generally slightly frusto-conical, the most proximal part being the narrowest. It is not excluded that the outer surface 13 could have a different taper towards the proximal end or be generally cylindrical. However, frusto-conical is preferred, *inter alia* because it provides greater strength against bending than a cylindrical shape.

At the most proximal end of the narrower part 10, i.e. at the righthand side of the bending section part 6 in Fig. 2, the outer diameter of the of the outer surface 13 is preferably adapted to match the inner diameter of the tubular main body 5 of the insertion tube 2.

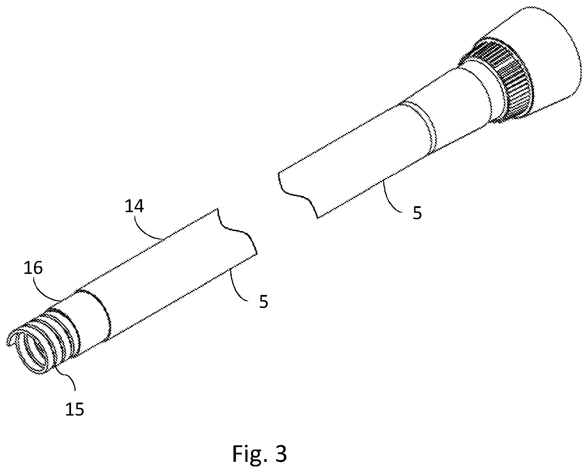

As can be seen in Fig. 3 the tubular main body 5 of the insertion tube 2 comprises least three layers, an outer air and/or watertight sheath 14 of a polymer material, an inner layer provided by a wound helical member 15 and an intermediate layer of a braided material 16. The wound helical member provides 15 the tubular main body 5 with compression resistance, preventing it largely from axial compression and radial compression, thus preventing it from kinking. The braided layer 16 on the other hand provides torsional resistance, so that a turning motion of the proximal end of the insertion tube 2 is transferred to the distal end of the insertion tube 2. The outer surface of the wound helical member 15 is preferably knurled with a pattern adapted to engage the pattern with which the braided layer 16 is braided. Please note that the braided layer 16 and the outer layer 14 have only been shortened in Fig. 3 for illustration purposes. Normally the longitudinal extension of the helical member 15, the braided layer 16 and the outer watertight layer 16 will be the same, i.e. all three extending all the way to the distal end of the tubular main body 5.

Since the inner diameter of the tubular main body 5, i.e. of the wound helical member 15 matches the outer diameter of the outer surface 13 at proximal end of the narrower part 10 bending section 6 it allows the bending section part 6 to be joined with the of the tubular main body 5 to form an insertion tube sub-assembly.

More specifically, the thread 11a-11e on the outer surface 13 may engage the gaps between the windings of the wound helical member 15. The bending section part 6 may thus be joined to the tubular main body 5 simply by screwing the external thread 11a-11e into the wound helical member 15. To control how far the bending section part 6 is screwed into the wound helical member 15 a stop is preferably provided, e.g. in the form of a discontinuity 17 in the diameter of the segment 7e where the narrower part 10 ends.

As mentioned, the narrower part 10 is preferably tapered, in particular having a generally frusto-conical outer surface 13. Thus, when the bending section part 6 is screwed into the wound helical member 15, the wound helical member 15 will be expanded slightly against its inherent spring forces, and against whatever counter-forces the braided layer 16 and the outer air and/or watertight layer 14 may provide. These forces which increase towards the distal end of the tubular main body 5 because of the increasing diameter of the outer surface 13, will aid in securing the joint between the bending section part 6 and the tubular main body 5.

Apart from that, the frusto-conical shape in itself increases the strength of the joint which is important because the joint is subject to substantial bending, compression and torsional forces, when the insertion tube is inserted through a tortuous part in the body of a patient to be treated, examined or goes through other medical procedures involving the endoscope.

For further securing the joint, glue may be added to the gaps between the windings of the wound helical member 15 during assembly. When, as preferred, the external thread 11a-11e is segmented, the glue will also enter and set in the gaps 12 between the individual segments 11a-11e, thereby securing the joint between the position of the tubular main body 5 and the bending section part 6. In particular, the set glue will provide bridges between individual windings of the wound helical member 15 in the gaps 12 between the individual segments 11a-11e, thereby form-locking the wound helical member 15 with respect to the individual segments 11a-11e, in turn improving pull strength and securing against mutual dislodging and rotation.

When the bending section part 6 has been joined with the insertion tube main body 5, an outer sheath may be added, so as to seal the central passage of the bending section against the environment. This outer sheath may evidently overlap the outer watertight layer 16 to further improve the sealing of the joint. A closed sealed conduit from the handle through the insertion tube to the most distal section 7a of the bending section is thus provide, so as to accommodate for wiring and tubes for e.g. working channels.

## Claims

1. An endoscope (1) comprising an insertion tube sub-assembly where the insertion tube sub-assembly comprises a bending section part (6) and a tubular main part (5) adapted to extend from an endoscope handle (3) towards the bending section part (6) at the distal end of the insertion tube (2), wherein the tubular main part (5) comprises a wound helical member (15), and
where the bending section part (6) comprises a number of articulated segments (7a-7e), and a connection part adapted for connecting the bending section part (6) to the tubular main part (5), the connection part comprising an external thread (11a-11e) adapted to engage the wound helical (15) member of the tubular main part (5), wherein
the external thread (11a-11e) comprises a number of individual thread segments (11a-11e) arranged with spaces (12) between the individual thread segments (11a-11e).

2. An endoscope (1) according to claim 1, comprising glue introduced in at least one of said spaces (12).

3. An endoscope (1) according to any one of the preceding claims, wherein the connection part comprises a generally frusto-conical outer surface (13) on which said external thread (11a-11e) is provided.

4. An endoscope (1) according to claim 1, wherein the bending section part (6) is a single-piece integrally moulded part.

5. A system comprising display unit (100) and an endoscope (1) according to any one of claims 1-4 connectable to said display unit (100).

## Patentansprüche

1. Endoskop (1), umfassend eine Einführrohr-Unterbaugruppe, wobei die Einführrohr-Unterbaugruppe einen Biegeabschnittteil (6) und einen röhrenförmigen Hauptteil (5) umfasst, der dazu ausgeführt ist, sich von einem Endoskopgriff (3) zu dem Biegeabschnittteil (6) hin an dem distalen Ende des Einführrohrs (2) zu erstrecken,
wobei der röhrenförmige Hauptteil (5) ein gewundenes spiralförmiges Element (15) umfasst und
wobei der Biegeabschnittteil (6) eine Anzahl von Gelenksegmenten (7a - 7e) und einen Verbindungsteil umfasst, der zum Verbinden des Biegeabschnittteils (6) mit dem röhrenförmigen Hauptteil (5) ausgeführt ist, wobei der Verbindungsteil ein Außengewinde (11a - 11e) umfasst, das dazu ausgeführt ist, das gewundene spiralförmige Element (15) des röhrenförmigen Hauptteils (5) in Eingriff zu nehmen, wobei
das Außengewinde (11a - 11e) eine Anzahl von einzelnen Gewindesegmenten (11a - 11e) umfasst, die mit Zwischenräumen (12) zwischen den einzelnen Gewindesegmenten (11a - 11e) angeordnet sind.

2. Endoskop (1) nach Anspruch 1, umfassend Klebstoff, der in mindestens einen der Zwischenräume (12) eingeführt wurde.

3. Endoskop (1) nach einem der vorhergehenden Ansprüche, wobei der Verbindungsteil eine allgemein kegelstumpfförmige Außenfläche (13) umfasst, auf der das Außengewinde (11a - 11e) vorgesehen ist.

4. Endoskop (1) nach Anspruch 1, wobei der Biegeabschnittteil (6) ein einteiliges, integral geformtes Teil ist.

5. System, umfassend eine Anzeigeeinheit (100) und ein mit der Anzeigeeinheit (100) verbindbares Endoskop (1) nach einem der Ansprüche 1 - 4.

## Revendications

1. Endoscope (1) comprenant un sous-ensemble tube d'insertion, le sous-ensemble tube d'insertion comprenant une partie de section de courbure (6) et une partie principale tubulaire (5) conçue pour s'étendre à partir d'une poignée d'endoscope (3) vers la partie de section de courbure (6) à l'extrémité distale du tube d'insertion (2), la partie principale tubulaire (5) comprenant un élément hélicoïdal enroulé (15), et
lorsque la partie de section de courbure (6) comprend un certain nombre de segments articulés (7a-7e), et une partie de connexion adaptée pour connecter la partie de section de courbure (6) à la partie principale tubulaire (5), la partie de connexion comprenant un filetage externe (11a-11e) adapté pour engager l'élément hélicoïdal enroulé (15) de la partie principale tubulaire (5)
le filetage externe (11a-11e) comprenant un certain nombre de segments de filetage individuels (11a-11e) disposés avec des espaces (12) entre les segments de filetage individuels (11a-11e).

2. Endoscope (1) selon la revendication 1, comprenant de la colle introduite dans au moins un desdits espaces (12).

3. Endoscope (1) selon l'une quelconque des revendications précédentes, la partie de connexion comprenant une surface extérieure généralement tronconique (13) sur laquelle ledit filetage externe (11a-11e) est prévu.

4. Endoscope (1) selon la revendication 1, la partie de section de flexion (6) étant une partie moulée d'une seule pièce.

5. Système comprenant une unité d'affichage (100) et un endoscope (1) selon l'une quelconque des revendications 1-4 pouvant être connecté à ladite unité d'affichage (100).
